# FASCICULE DE BREVET EUROPEEN

(11) **EP 2 976 314 B1**
(45) Date de publication et mention de la délivrance du brevet: **16.12.2020**
(21) Numéro de dépôt: 14716886.8
(22) Date de dépôt: 18.03.2014
(51) Int. Cl.: C07C 7/144, C07C 253/30, C07C 255/23, C07C 11/04, C07C 253/34, B01D 61/36, B01D 61/00

(54) **PROCÉDÉ DE MÉTATHÈSE COMPRENANT L'EXTRACTION DE L'ÉTHYLÈNE FORME AU MOYEN D'UNE MEMBRANE**
METATHESEVERFAHREN MIT ETHYLENEXTRAKTION MITTELS EINER MEMBRAN
METATHESIS PROCESS COMPRISING THE EXTRACTION OF THE ETHYLENE FORMED BY MEANS OF A MEMBRANE

(30) Priorité: 19.03.2013 FR 1352413
(43) Date de publication de la demande: 27.01.2016
(73) Titulaire: Arkema France, 92700 Colombes (FR)
(72) Inventeur: DUBOIS, Jean-Luc, 69390 Millery (FR); COUTURIER, Jean-Luc, 69006 Lyon (FR)
(74) Mandataire: Arkema Patent
(86) Numéro de dépôt international: PCT/FR2014/050628
(87) Numéro de publication internationale: WO 2014/147337

(56) Documents cités:
- WO-A1-2010/055273
- WO-A1-2013/034690
- WO-A2-01/83097
- DE-A1- 19 642 278
- DJIGOUE G B ET AL: "Improving the selectivity for the synthesis of two renewable platform chemicals via olefin metathesis", APPLIED CATALYSIS A: GENERAL, ELSEVIER SCIENCE, AMSTERDAM, NL, vol. 368, no. 1-2, 31 octobre 2009 (2009-10-31), pages 158-162, XP026641994, ISSN: 0926-860X, DOI: 10.1016/J.APCATA.2009.08.025 [extrait le 2009-08-25]

## Description

Le travail qui a conduit à cette invention a reçu un financement de la part de l'Union Européenne dans le cadre du 7^{ème} Programme Cadre (FP7/2007-2013) sous le numéro de projet N°241718 EUROBIOREF.

### DOMAINE DE L'INVENTION

L'invention concerne un procédé de métathèse comprenant l'extraction de l'éthylène formé du milieu réactionnel au moyen d'au moins une membrane perméable aux gaz et imperméable aux liquides.

### ARRIERE-PLAN TECHNIQUE

La réaction de métathèse croisée est une réaction équilibrée selon le schéma A+B ⇔ C+D, dans laquelle la réaction inverse se produit à l'approche de l'équilibre. En général, pour obtenir la synthèse du produit final recherché, C ou D dans le schéma, on déplace l'équilibre de la réaction soit en fournissant un réactif en excès par rapport à l'autre, soit en soutirant l'un des produits synthétisés au fur et à mesure de sa formation. Une telle réaction de métathèse est décrite dans les documents suivants. WO 2010/055273 décrit une réaction de métathèse entre deux composés alpha-oléfiniques, un nitrile et l'acrylate de méthyle. WO 01/83097 décrit une réaction d'homométathèse d'une oléfine allylique.

La voie qui consiste à fournir un réactif en excès a ses limites. En effet, la métathèse est une réaction catalytique dont les catalyseurs efficaces pour la réaction de métathèse croisée favorisent également la réaction d'homo-métathèse de chacun des composés A et B du schéma ci-dessus qui accompagne toujours la réaction principale. Ceci conduit à des mélanges dont la séparation est difficile au sein du réacteur.

Un autre problème rencontré dans les réactions de métathèse est la désactivation ou l'inhibition du catalyseur sous l'action de certains réactifs et produits de métathèse, catalyseur qui - il faut le souligner - est coûteux. Il serait donc souhaitable pour éviter une désactivation/inhibition rapide du catalyseur de travailler avec de faibles concentrations de ces réactifs et/ou produits.

La réaction de métathèse est conduite en phase liquide en présence de catalyseurs appartenant généralement à une famille de composés du ruthénium qui sera décrite plus loin. La séparation totale en continu des composés C ou D sous forme liquide dans les conditions de la réaction est en pratique impossible au sein du réacteur. En effet, les produits sont présents sous forme liquide et/ou soluble dans le milieu réactionnel et leur point d'ébullition est incompatible avec le domaine de stabilité du catalyseur, ce qui ne permet pas d'envisager une distillation en continu. Dans un certain nombre de cas, les propriétés physicochimiques des réactifs A ou B et des produits C ou D sont relativement proches. Il est donc préférable de travailler avec une conversion élevée des réactifs pour ne pas rencontrer de problèmes de séparation. Dans ce contexte, les industriels choisissent de mener la réaction de métathèse avec des co-réactifs A et B susceptibles de former par métathèse des composés oléfiniques gazeux pouvant être extraits du milieu réactionnel par distillation. Ces composés oléfiniques sont par exemple l'éthylène, le propylène ou les butènes, avec une préférence pour l'éthylène dont la température d'ébullition est la plus basse.

Quand l'un des composés C ou D est un composé léger, il peut être éliminé en partie par « distillation continue » soit en diminuant la pression totale (en travaillant sous vide partiel), cependant cette solution présente aussi comme risque une introduction accidentelle d'oxygène dans le réacteur, soit en chauffant le milieu réactionnel pour augmenter sa tension de vapeur. Cependant cette dernière solution réduit la durée de vie du catalyseur par désactivation thermique, réduit la sélectivité de la métathèse croisée par rapport aux réactions d'homo-métathèse et a tendance à promouvoir les réactions d'isomérisation par déplacement de la double liaison. On recherche donc une solution technique qui soit de préférence aussi bien applicable à basse température qu'à haute température.

Il est connu, voir à ce sujet la publication de Jim Patel et al. intitulé « High conversion and productive catalyst turnovers in cross-metathesis reactions of natural oils with 2-butene » paru dans Green Chem., 2006, 8, pp 450-454*,* que dans le cas où la réaction de métathèse croisée est réalisée en présence d'a-oléfines comme réactif ou comme produit, le catalyseur peut perdre rapidement au cours du temps de son activité. Dans le cas des réactions de métathèse impliquant de l'éthylène, le catalyseur subit des transformations qui inhibent fortement la métathèse. On pense peut-être pouvoir attribuer cette inhibition à la formation d'une espèce avec le catalyseur, par exemple de type Ru=CH₂, de sorte que le catalyseur se désactive très rapidement, conduisant ainsi à une perte importante de catalyseur. Pour résoudre ce problème, il est nécessaire de limiter la présence dans la phase liquide d'espèces à double(s) liaison(s) terminale(s), d'éthylène notamment, dissous dans le milieu réactionnel.

Lorsque les réactifs eux-mêmes ont des doubles liaisons terminales, la seule possibilité est d'éliminer l'éthylène formé du milieu réactionnel liquide. L'éthylène est un gaz, qui s'élimine naturellement du liquide réactionnel par distillation du fait de son faible point d'ébullition. Cependant sa solubilité dans le milieu réactionnel liquide est fonction de la température et est d'autant plus grande que la température de réaction est faible. On pourrait envisager de conduire la réaction à plus haute température pour diminuer sa solubilité dans le milieu. Cependant, les températures élevées imposent d'autres conditions opératoires plus coûteuses en matière de solvant, de pression opératoire, de dégradation du catalyseur, de sélectivité de la réaction, etc.

Les solutions existantes pour éliminer l'éthylène consistent soit à entraîner l'éthylène par distillation à des températures supérieures à 120°C ce qui a tendance à provoquer la dégradation thermique du catalyseur, soit à travailler avec un milieu réactionnel sous vide partiel pour éliminer plus rapidement l'éthylène, mais cette solution n'est pas préférée industriellement car le vide augmente considérablement le coût du procédé.

Un compromis doit donc être trouvé entre i) une température élevée qui permet d'avoir de plus faibles solubilités de l'éthylène et un plus fort entraînement de l'éthylène gazeux et ii) une température suffisamment basse pour limiter la désactivation thermique du catalyseur.

La présente invention a donc pour but de fournir un procédé de métathèse dans lequel l'éthylène est éliminé le plus rapidement possible lors de la réaction de métathèse. En particulier, la présente invention a pour but de fournir un tel procédé susceptible de fonctionner à basse température comme à haute température et permettant d'empêcher l'inhibition de la réaction de métathèse.

La Demanderesse a maintenant trouvé, de manière totalement surprenante et inattendue, qu'en faisant circuler le milieu réactionnel de la métathèse sur un système membranaire perméable aux gaz permettait de soutirer l'éthylène formé et en partie dissous dans la phase liquide du milieu réactionnel, de limiter l'inhibition de la réaction et même de diminuer les quantités nécessaires de catalyseur de métathèse tout en améliorant les performances de la réaction de métathèse.

Il est connu d'utiliser des membranes ou plus généralement des systèmes membranaires pour éliminer des gaz d'un liquide ou inversement pour gazéifier un liquide. On peut à ce sujet se référer à l'article de Miroslav Stanojevic et al. intitulé « Review of membrane contactors designs and applications of différent modules in industry » publié dans FME Transactions Vol. 31, N°2, pp 91-98 (2003*).* A côté des applications classiques de dégazage ou de gazéification des systèmes aqueux, on connait comme autres applications industrielles de ces techniques : le dégazage de fluides organiques diélectriques ou hydrauliques, le dégazage des encres pour imprimantes à jet d'encre et toute une gamme de séparations par membranes, poreuses ou denses dans l'industrie alimentaire.

Le brevet US 6,217,634 décrit l'utilisation de systèmes membranaires pour éliminer les gaz formés lors de la décomposition des fluides diélectriques qui peuvent provoquer la destruction de transformateurs. Cette méthode est généralement utilisée en contrôle du comportement des transformateurs en vue d'adapter le traitement industriel d'élimination des gaz légers par extraction sous vide.

Le brevet US 8,205,977 décrit une méthode similaire pour le dégazage d'encres pour imprimantes à jet d'encre pour éviter les problèmes d'absence de pixels sur l'impression.

Le brevet US 6,402,810 décrit un procédé de dégazage et de déshydratation par membrane de fluides hydrauliques organiques.

Le brevet US 6,790,262 décrit un dispositif membranaire de dégazage de liquides et plus particulièrement d'encre.

L'art antérieur précité est uniquement dédié à la purification de liquides pour en extraire des impuretés généralement gazeuses. Le problème à résoudre par le procédé de l'invention n'est pas d'éliminer des impuretés, mais d'extraire du milieu réactionnel en cours de réaction, tout ou partie de l'éthylène produit pour améliorer les performances du procédé. La solution de ce problème passe par l'utilisation d'un système membranaire implanté dans le dispositif réactionnel de métathèse croisée. La plupart des membranes utilisées dans les applications visées ci-dessus dans l'art antérieur, s'avèrent inadaptées et ne répondent pas au nouveau problème posé par le procédé de l'invention pour de multiples raisons telles que :
i) résistance à la diffusion des gaz par des membranes denses uniques et trop épaisses,
ii) perméabilité insuffisante aux gaz susceptible de provoquer des « bouchages », ralentissant la réaction, voire la bloquant,
iii) stabilité chimique insuffisante,
iv) gonflement des membranes à température élevée,
v) incompatibilité chimique des membranes avec les solvants, voire avec les réactifs ou les produits eux-mêmes, se traduisant par des problèmes de dégradation et de vieillissement accéléré des membranes,
vi) inhibition du catalyseur entraînant de faibles taux de conversion de réaction, etc. Les techniques membranaires existantes ne permettent pas de résoudre de manière satisfaisante les problèmes précités, ne serait-ce parce que les conditions opératoires et les divers produits chimiques diffèrent totalement.

La présente invention a donc pour but de fournir un nouveau procédé de métathèse mettant en oeuvre un système d'élimination d'éthylène, qui présente à la fois une perméabilité sélective adaptée, une bonne tenue en température et une stabilité chimique vis-à-vis des divers produits présents dans le milieu réactionnel.

### DESCRIPTION DE L'INVENTION

L'invention est maintenant décrite plus en détail et de façon non limitative dans la description qui suit.

La présente invention a donc pour objet un procédé de métathèse de deux composés α-oléfiniques, caractérisé en ce qu'il comprend l'extraction de l'éthylène formé du milieu réactionnel au moyen d'au moins une membrane perméable aux gaz et imperméable aux liquides.

Le procédé de l'invention concerne plus particulièrement la métathèse de deux composés α-oléfiniques et est caractérisé en ce qu'il est conduit dans un dispositif réactionnel comportant deux zones séparées par au moins une membrane perméable aux gaz et imperméable aux liquides :
- une première zone, alimentée en réactifs et catalyseur, dans laquelle la réaction de métathèse en phase liquide est initiée et le milieu réactionnel liquide est mis en circulation au contact de la paroi constituée par la membrane ; et
- une seconde zone, alimentée en un flux gazeux inerte vis-à-vis de la membrane et des constituants du milieu réactionnel de la première zone, éventuellement sous une pression légèrement inférieure à celle régnant dans la première zone ou sous la forme d'un balayage par un courant gazeux à débit suffisant, de façon à entraîner la migration par diffusion de l'éthylène dissous dans le milieu réactionnel de la première zone à la seconde zone.

Le procédé de l'invention s'applique à une réaction de métathèse entraînant la formation d'éthylène. Le procédé de l'invention s'applique notamment à la métathèse croisée de deux composés α-oléfiniques, fonctionnalisés ou non.

Le procédé de l'invention s'applique plus particulièrement à la métathèse croisée entre un composé fonctionnel ω-insaturé et une α-oléfine légère fonctionnalisée ou non. Enfin, le procédé de l'invention est également utilisable dans la synthèse d'un composé bi-fonctionnel symétrique par homo-métathèse d'un composé α-oléfinique fonctionnel.

Dans la présente description on entend au sens de l'invention par :
- « composé α-oléfinique », une molécule, fonctionnalisée ou non, ayant une double liaison terminale de formule R₁R₂C=CH₂ dans laquelle R₁ et R₂, identiques ou différents, sont, H, un radical alkyle, alkényle ou aryle comportant de 1 à 14 atomes de carbone et comportant éventuellement une fonction, telle que : acide, ester, nitrile, alcool ou aldéhyde ..., étant précisé que R₁ et R₂ ne sont pas simultanément H ;
- « α-oléfine légère » (ou oléfine légère) une oléfine comportant de 3 à 5, de préférence de 3 à 4, atomes de carbone et éventuellement porteuses d'une ou plusieurs fonctions acide, ester ou nitrile telles que l'acrylonitrile, les acrylates d'alkyles, etc ;
- « dispositif réactionnel » l'ensemble des éléments, quelle que soit leur localisation, dans lesquels circule en circuit fermé le milieu réactionnel liquide.

Le procédé de l'invention permet notamment la synthèse d'acides/esters gras mono- ou di-fonctionnels. La matière première utilisée dans le procédé de l'invention est avantageusement un acide/ester/nitrile gras insaturé d'origine naturelle dont la ou les double(s) liaison(s) est (sont) en principe localisées en position 6, 9, 10, 11, 12, 13 ou 15. Il est nécessaire de modifier cet ester/acide/nitrile gras en le soumettant notamment à une réaction préalable d'éthénolyse ou de craquage thermique (pyrolyse) permettant de conduire à un composé ω-insaturé acide/ester/nitrile gras de formule CH₂=CH-(CH₂)ₙ-R dans laquelle R est COOH, COOR₃ ou CN, R₃ étant un radical alkyle comprenant de 1 à 11 atomes de carbone et n un nombre entier compris dans la gamme de 3 à 13.

La réaction de métathèse croisée est conduite en milieu liquide en présence d'un catalyseur dans les conditions opératoires suivantes. La température est généralement comprise dans la gamme de 20 à 160 °C et de préférence de 20 à 120 °C. La pression est généralement comprise dans la gamme de 1 à 30 bars. La réaction est de préférence conduite à basse pression comprise dans la gamme de 1 à 10 bars et de façon plus préférée à la pression atmosphérique lorsque la température d'ébullition des réactifs mis en jeu le permet. En effet, dans la mesure où l'on vise un dégagement d'éthylène, il est avantageux de travailler à basse pression, pression atmosphérique de préférence. La réaction peut être conduite sans solvant ou en présence d'au moins un solvant, tel que le toluène, les xylènes ou le dichlorométhane par exemple. La réaction est de préférence conduite sans solvant.

La première zone est de préférence pourvue de moyens d'extraction de la phase gazeuse formée, au-dessus du milieu réactionnel, l'éthylène produit notamment, ainsi que de moyens d'extraction en fin de réaction dudit milieu réactionnel d'une part. La deuxième zone est pourvue de moyens d'extraction du flux gazeux enrichi en éthylène d'autre part.

La présente invention permet de surmonter les inconvénients des procédés de métathèse de l'art antérieur en permettant l'extraction de l'éthylène formé au cours de la réaction de métathèse et notamment celui dissous dans la phase liquide du milieu réactionnel. Elle fournit plus particulièrement un procédé de métathèse croisée d'un composé α-oléfinique fonctionnel avec une oléfine légère telle que le propylène ou les butènes, ou avec une oléfine fonctionnalisée telle que l'acrylonitrile ou l'acrylate de méthyle, conduisant directement à des taux de conversion, jusque là impossibles à atteindre avec les procédés de métathèse conventionnels.

De préférence le procédé de l'invention comprend l'élimination en continu de l'éthylène d'une part présent en phase gazeuse, et d'autre part l'élimination simultanée de l'éthylène dissous dans le milieu réactionnel liquide par diffusion à travers la membrane perméable aux seuls gaz. Cette double élimination permet d'augmenter le rendement de la réaction.

Avantageusement, le procédé de l'invention est conduit dans un dispositif réactionnel comprenant au moins deux zones séparées par une membrane perméable aux gaz et imperméable aux liquides.

Sur le plan industriel on utilise de préférence pour la mise en oeuvre du procédé une pluralité de membranes ou de systèmes membranaires, définissant par exemple alternativement une zone réactionnelle et une zone d'extraction de l'éthylène car il est important d'augmenter les surfaces de contact lors de la mise en oeuvre du procédé.

Il existe deux types principaux de membranes, les membranes poreuses ou microporeuses d'une part utilisées pour divers types de filtration et les membranes denses dont la perméabilité sélective est fondée sur un mécanisme de solution-diffusion. Dans le procédé de l'invention on utilise au moins une membrane dense pour la séparation et l'extraction sélective de l'éthylène

Avantageusement, la membrane utilisée selon l'invention est formée au moins partiellement de polymère. L'imperméabilité aux liquides implique l'utilisation d'au moins un polymère homogène dit « dense » qui doit aussi permettre la diffusion de l'éthylène produit par la réaction. Les membranes en polymères denses peuvent constituer la totalité de la membrane (membrane uni-couche), une partie seulement de la membrane dans le cas où les polymères denses sont associés à d'autres matériaux (membrane composite), ou encore peuvent être supportées sous la forme d'un film mince déposé sur une membrane support poreuse qui assure la tenue mécanique du système membranaire (membrane asymétrique). Dans ce dernier cas, la membrane dense est beaucoup moins épaisse ce qui permet aussi de réduire sa résistance à la diffusion des gaz. Les membranes support poreuses comportent des pores dont la taille est généralement comprise dans la gamme de 0,01 à 50 µm.

Toute forme de membrane est utilisable, mais elle répond de préférence cumulativement à plusieurs impératifs techniques :
- être imperméable aux liquides du milieu réactionnel mais présenter une bonne perméabilité aux composés gazeux issus de la réaction ;
- présenter une tenue mécanique suffisante pour tenir la différence de pression entre la zone liquide et la zone gazeuse,
- être inerte vis-à-vis des catalyseurs, des réactifs et produits de la réaction et corrélativement ne pas être sensible à une action physique ou chimique de ces derniers qui pourrait entraîner une perte de performance,
- avoir une bonne tenue en température pour supporter les variations au cours du processus réactionnel.

La tenue mécanique de la membrane est directement liée à son épaisseur. L'épaisseur des membranes ayant une tenue mécanique convenable est généralement comprise dans la gamme de 5 à 1000 µm, de préférence de 5 à 700 µm, de préférence de 5 à 300 µm, et de préférence de 10 à 70 µm. Lorsqu'une membrane asymétrique est utilisée, l'épaisseur totale est de préférence dans la gamme de 5 à 300 µm et de préférence de 10 à 70 µm et l'épaisseur de la membrane dense est généralement comprise dans la gamme de 0,01 à 10 µm, et de préférence de 0,1 à 1 µm.

Tout en respectant les critères précédents, le choix de la nature de la membrane dans le procédé de l'invention est fonction du milieu réactionnel, des catalyseurs, réactifs, produits, et solvant le cas échéant, afin d'éviter toute interaction avec la membrane. Les conséquences les plus courantes, outre une transformation des produits formés au sein du milieu réactionnel liquide, sont une altération de la membrane pouvant gêner, voire bloquer, la réaction de métathèse dans le milieu liquide.

Selon un premier mode de réalisation, le procédé de l'invention utilise au moins une membrane polymère dense uni-couche, c'est-à-dire constituée au moins partiellement d'un polymère homogène non poreux, perméable à l'éthylène mais imperméable aux liquides.

Selon un deuxième mode de réalisation alternatif ou pouvant être combiné avec le précédent, le procédé de l'invention utilise au moins une membrane polymère asymétrique comportant une couche (de préférence de polymère) poreuse perméable aux gaz et aux liquides, recouverte d'une couche de membrane polymère dense, continue (non poreuse), perméable à l'éthylène mais imperméable aux liquides, de préférence d'épaisseur plus fine que celle de la couche de poreuse. Dans cette seconde configuration, le gaz, du fait de l'épaisseur moindre de la couche de polymère dense, rencontre moins de résistance à la diffusion ce qui permet d'accélérer le processus de diffusion. Ce type de membrane asymétrique mixte est donc préféré pour le procédé de l'invention.

La forme de la membrane utilisée dans le procédé de la présente invention est également choisie en fonction du type de réacteur utilisé et de l'objectif industriel visé. La membrane peut se présenter sous la forme d'un film plan, sous la forme d'un film tubulaire ou cylindrique ou celle d'une fibre creuse, de dimensions adaptées.

La fibre creuse unitaire (ou le film tubulaire) a généralement un diamètre extérieur compris dans la gamme de 50 à 1000 µm, de préférence de 150 à 700 µm et de manière plus préférée de 250 à 300 µm. Le diamètre intérieur de la fibre est généralement compris dans la gamme de 30 à 300 µm. Dans le cas de la fibre creuse ou du film tubulaire, l'épaisseur de la membrane est de préférence comprise dans la gamme de 10 à 70 µm et celle de la membrane dense dans le cas d'une membrane asymétrique comprise de préférence dans la gamme de 0,01 et 10 µm. On nomme ci-après « module » ou « module membranaire » l'ensemble formé par une ou plusieurs membranes. L'utilisation de module(s) à fibres creuses et à un degré moindre celle de module(s) à film tubulaire ou cylindrique constituent une solution particulièrement avantageuse par rapport aux membranes planes car elles permettent d'augmenter le ratio surface d'échange sur volume de milieu réactionnel traité, d'accélérer l'extraction de l'éthylène formé et donc d'augmenter encore l'efficacité du procédé de l'invention. Le procédé utilise donc de préférence une ou plusieurs membranes sous la forme de fibres creuses, permettant d'avoir un plus grand ratio de surface d'échange par unité de volume de liquide traité, que dans le cas de la membrane plane.

Selon un premier mode de mise en œuvre du procédé de l'invention un module sous la forme d'un faisceau de fibres creuses est immergé dans le réacteur où est conduite la réaction de métathèse et l'éthylène est soutiré et extrait par balayage gazeux, de préférence à l'intérieur de la fibre. Le cas échéant, une pression légèrement inférieure à celle du réacteur est appliquée au balayage. Ceci suppose naturellement que les fibres utilisées aient une tenue thermique/mécanique compatible avec les conditions opératoires de température et de pression de la métathèse. Il est possible de procéder de manière inverse, à savoir de conduire la réaction à l'intérieur de chaque fibre et d'effectuer l'extraction de l'éthylène au moyen du flux gazeux de l'espace environnant les fibres (autour des fibres) auquel on applique cas échéant un différentiel de pression. Cependant une telle solution est moins préférée notamment à cause du possible problème de perte de charge à l'intérieur de la fibre.

Selon un deuxième mode de réalisation du procédé de l'invention, la réaction de métathèse est conduite dans un réacteur qui est connecté (par exemple dans sa partie basse) à un dispositif de séparation membranaire comportant au moins un module d'extraction de l'éthylène. Une partie du flux liquide du milieu réactionnel est ainsi adressée au dispositif de séparation membranaire, par l'intermédiaire d'une pompe par exemple. Le flux liquide sortant dudit dispositif est retourné dans le réacteur pour la poursuite de la réaction. Ce type de configuration présente l'avantage de pouvoir maîtriser la température du milieu réactionnel liquide adressé au dispositif de séparation membranaire. En effet, dans le cas où les conditions de température de la réaction sont incompatibles avec une bonne efficacité de l'extraction, par exemple une température trop élevée, on implante sur le circuit deux échangeurs thermiques, le premier en amont du séparateur pour abaisser la température du flux liquide à son seuil de fonctionnement et le second en aval pour remonter la température du flux liquide au niveau de celle du milieu réactionnel du réacteur avant son recyclage.

Dans le second mode de réalisation, le milieu réactionnel liquide est adressé au dispositif de séparation membranaire. Le dispositif est avantageusement choisi parmi plusieurs types de modules, donc les plus adaptés sont notamment le module plan, le module spirale, ou encore le module à fibre creuse.

Selon encore un autre mode de réalisation du procédé de l'invention mettant en oeuvre au moins un (module à) film tubulaire (ou bien un module à fibre creuse), on fait circuler le milieu réactionnel à l'intérieur du film (ou des fibres) au sein du module dans lequel circule une phase gazeuse devant être enrichie en éthylène. Il est également possible d'opérer à l'inverse, c'est-à-dire de faire circuler le gaz d'extraction à l'intérieur des fibres et le milieu réactionnel dans l'espace libre restant du module.

Au sein du module, l'extraction de l'éthylène pourra s'effectuer par écoulement à co-courant, à contre-courant ou à courant croisé, de préférence à courant croisé.

Des modules de ce type permettent d'augmenter de manière importante la surface d'échange au niveau des membranes entre le flux d'alimentation et celui d'extraction.

Un des critères de choix de polymère(s) pour la membrane dense, utilisée soit seule en uni-couche, soit sous forme asymétrique associée à une membrane poreuse, est son caractère oléophobe. En outre, elle est de préférence résistante à la chaleur afin de pouvoir être utilisée aux températures de mise en oeuvre de la réaction de métathèse, sans avoir à appliquer au milieu réactionnel des échanges thermiques. Enfin, il importe de vérifier son inertie physique et chimique vis-à-vis des réactifs, produits ou solvants intervenant dans la réaction de métathèse.

A titre d'exemples de polymères pouvant entrer dans la composition des membranes utilisables dans le procédé de l'invention, on peut citer les polyoléfines telles que le polyéthylène, le polypropylène, le poly-3-méthylbutène-1 et le poly-4-méthylpentène-1 (PMP); les polymères vinyliques tels que le polystyrène, le PMMA (polyméthyl-méthacrylate) ; les polysulfones ; les polymères fluorés ou chlorés tels que le polyvinylidène fluoré (PVDF) , le polytétrafluoroéthylène (PTFE), les copolymères fluorovinyléthylène / tétrafluoroéthylène, le perfluoro-2,2-diméthyl-1,3-dioxole (PDD), le perfluoroalkoxy (PFA), le polychloroprène (PCP) ; les polyamides tels que le nylon 6, le nylon 66 et le nylon 12 ; les polyesters tels que le polyéthylènetéréphtalate, le polybutènetéréphtalate et le polyéthylène-2,6-naphtalate ; les polycarbonates tels que le poly-4,4'-dihydroxydiphénil-2,2-propane carbonate ; les polyéthers tels que le poly-oxyméthylène et le polyméthylène sulfure ; les poly-phénylènes chalcogénures tels que les polythioéther, polyphénylène oxyde et polyphénylène sulfure ; les polyéther éther cétones (PEEK), les polyéther cétone cétone (PEKK), les silicones tels que le polyvinyltriméthylsiloxane (PVTMS), le polydiméthylsiloxane (PDMS ), le perfluoroalkoxy (PFA), le polychloroprène (PCP).

Parmi les polymères cités ci-dessus, le perfluoro-2,2-diméthyl-1,3-dioxole (PDD), le poly-tétrafluoro-éthylène (PTFE) ou le perfluoroalkoxy (PFA), et leurs mélanges, sont préférés pour la composition de la membrane selon le procédé de l'invention. Des fibres creuses particulièrement appropriées pour l'invention peuvent être fabriquées en utilisant, associés à la membrane dense, divers matériaux poreux, tels que le polypropylène, le polyfluorure de vinylidène (PVDF), le polysulfone. A titre d'exemple, l'intérieur et/ou l'extérieur des fibres creuses est revêtu d'une mince couche dense dont l'épaisseur peut aller de 0,01 à 10 µm d'un copolymère amorphe de perfluoro-2,2-diméthyl-1,3-dioxole (PDD) et de quantités variables de tétrafluoroéthylène (TFE). D'autres polymères fluorés peuvent également être utilisés avec succès. Par exemple, le TEFLON® AF 2400 et le TEFLON® AF 1600 (El du Pont de Nemours and Co) sont deux copolymères bien adaptés au procédé de l'invention. Cette couche fait barrière au milieu réactionnel organique, est de bonne tenue mécanique, tout en étant suffisamment perméable à l'éthylène pour permettre sa diffusion rapide vers la zone gazeuse.

La réaction de métathèse est conduite en présence d'au moins un catalyseur de métathèse. Ces catalyseurs sont bien connus et il en existe toute une gamme. On peut citer par exemple les complexes au tungstène développés par Schrock et al (J. Am. Chem. Soc. 108:2771, 1986) ou Basset et al. (Angew. Chem., Ed. Engl. 31:628, 1992). Plus récemment, sont apparus les catalyseurs dits de Grubbs (voir Grubbs et al., Angew. Chem., Ed. Engl. 34 :2039, 1995 et Organic Letters 1:953, 1999) qui sont des complexes ruthénium-benzylidène opérant en catalyse homogène. D'autres travaux ont été conduits pour la réalisation de catalyseurs immobilisés, c'est-à-dire de catalyseurs dont le principe actif est celui du catalyseur homogène, notamment les complexes ruthénium-carbène, immobilisés sur un support inactif.

Le procédé selon l'invention utilise avantageusement au moins un catalyseur de métathèse de type ruthénium-carbène. Ledit catalyseur ruthénium-carbène est choisi de préférence parmi les catalyseurs chargés ou non-chargés de formule générale :

(X₁)ₐ(X₂)_{b}Ru(carbène C)(L₁)_{c}(L₂)_{d}(L₃)ₑ

dans laquelle :
- a, b, c, d et e sont des nombres entiers, identiques ou différents, avec a et b égaux à 0, 1 ou 2 ; c, d et e égaux à 0, 1, 2, 3 ou 4;
- X₁ et X₂, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés ; X₁ ou X₂ peuvent être liés à L₁ ou L₂ ou au carbène C de façon à former un ligand bidenté ou chélate sur le ruthénium ; et
- L₁, L₂ et L₃ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique ; L₁, L₂ ou L₃ peuvent être liés au carbène C de façon à former un ligand bidenté ou chélate, ou tridenté.

Le carbène C est représenté par la formule générale : CR₁R₂ pour laquelle R₁ et R₂ sont des groupes identiques ou différents tels que l'hydrogène ou tout autre groupe hydrocarboné, fonctionnalisé ou non, de type saturé, insaturé, cyclique, aromatique, branché et/ou linéaire. A titre d'exemples, on pourra citer les complexes du ruthénium alkylidènes, benzylidène, benzylidène éther, ou cumylènes tels que les vinylidènes Ru=C=CHR ou allénylidènes Ru=C=C=CR₁R₂ ou indénylidènes.

Un groupe fonctionnel (permettant d'améliorer la rétention du complexe du ruthénium dans un liquide ionique) peut être greffé sur au moins l'un des ligands X₁, X₂, L₁, L₂, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

Le catalyseur de métathèse peut être éventuellement hétérogénéisé sur un support afin de faciliter sa récupération/recyclage.

Les catalyseurs de métathèse croisée du procédé de l'invention sont de préférence des carbènes du ruthénium décrits par exemple dans Aldrichimica Acta, vol 40, n°2, 2007, p.45-52.

Des exemples de tels catalyseurs sont les catalyseurs de Grubbs, les catalyseurs Hoveyda-Grubbs, les catalyseurs Piers-Grubbs, et autres catalyseurs de métathèse du même type, qu'ils soient dits de « 1^{ère} génération », « 2^{ème} génération » ou de « 3^{ème} génération ».

Les catalyseurs de Grubbs sont basés sur un atome de ruthénium entouré par 5 ligands :
- 2 ligands anioniques, tels que des halogénures ;
- 2 ligands donneurs d'électrons, tels que les tri-alkyl-phosphines, ou les carbènes N-hétérocycliques saturés (appelés ligands NHC) ;
- un groupement alkylidène, tels que des groupements méthylènes =CR₂ substitués ou non.

On classe ces catalyseurs de métathèse en deux catégories, suivant la nature de leurs ligands L donneurs d'électrons :
- ceux qui contiennent deux ligands phosphine (et pas de ligand NHC saturé), développés en premier, sont des catalyseurs de type 1^{ère} génération ;
- ceux qui contiennent un ligand NHC saturé (carbène hétérocyclique) sont des catalyseurs de type 2^{ème} génération.

Un type de catalyseur dit « Hoveyda-Grubbs » contient parmi les ligands donneurs d'électrons, un ligand chélatant benzylidène-éther, et soit une phosphine (1^{ère} génération) soit un ligand NHC saturé (2^{ème} génération), le plus souvent substitué par des phényles généralement substitués par des groupements mésityles (Mes) ou bien par des groupements isopropyles (iPr).

Un autre type de catalyseur dit « Piers-Grubbs », forme un complexe cationique à quatre ligands qui ne nécessite pas la dissociation d'un ligand avant la réaction. D'autres types de catalyseurs sont les catalyseurs « Umicore », « Zanan », « Grela ».

De manière générale, le choix du catalyseur dépend de la réaction considérée. Selon un mode de réalisation avantageux, le catalyseur est dépourvu de phosphine. Des catalyseurs préférés sont les catalyseurs suivants :
(1) Le catalyseur désigné par « Hoveyda-Grubbs 2 », de formule suivante : 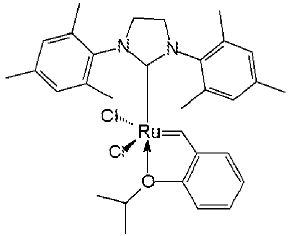
(2) Le catalyseur désigné par « M51 », de formule suivante : 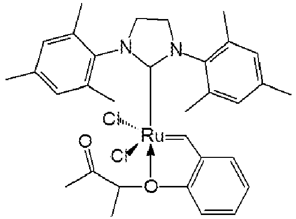
(3) Le catalyseur désigné par « M71-SIPr », de formule suivante : 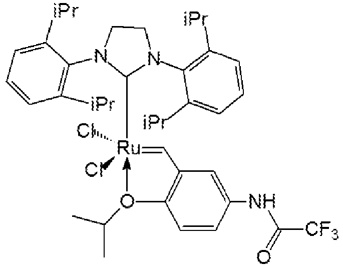
(4) Le catalyseur désigné par « M71-SIMes », de formule suivante : 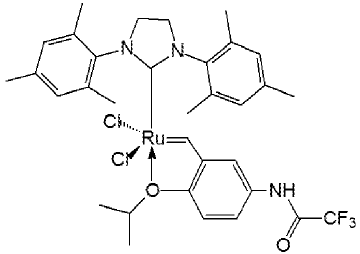
(5) Le catalyseur désigné par « M72-SIPr », de formule suivante : 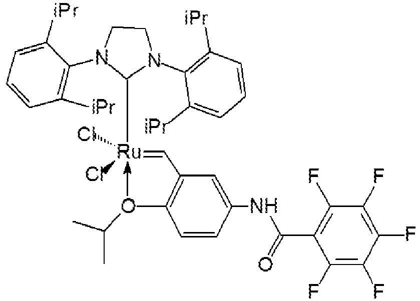
(6) Le catalyseur désigné par « M73-SIPr », de formule suivante : 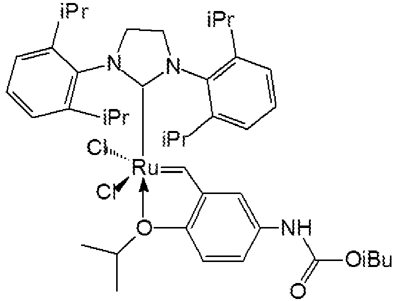
(7) Le catalyseur désigné par « M74-SIPr », de formule suivante : 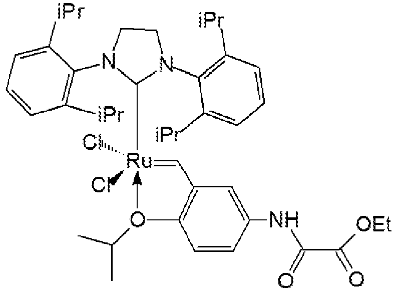
(8) Le catalyseur désigné par « Nitro-Grela-SIMes », de formule suivante : 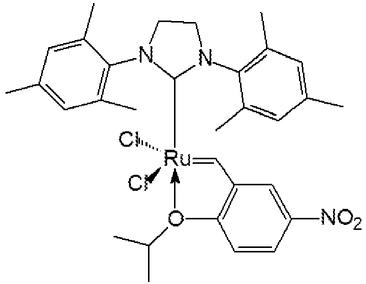
(9) Le catalyseur désigné par « Nitro-Grela-SIPr », de formule suivante : 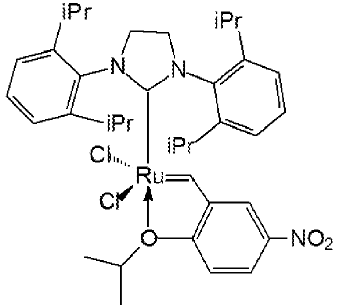
(10) Le catalyseur désigné par « Apeiron AS2034 », de formule suivante : 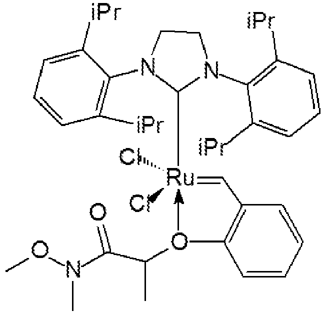
(11) Le catalyseur désigné par « Zannan 44-0082 (Strem) », de formule suivante : 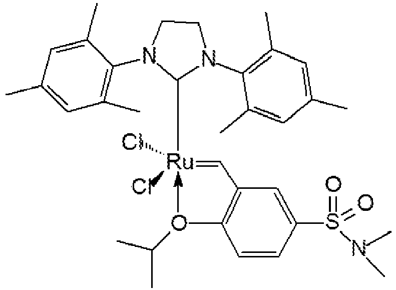
(12) Le catalyseur désigné par « M831-SIPr », de formule suivante : 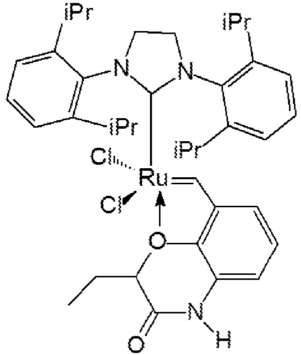
(13) Le catalyseur désigné par « M832-SIPr », de formule suivante : 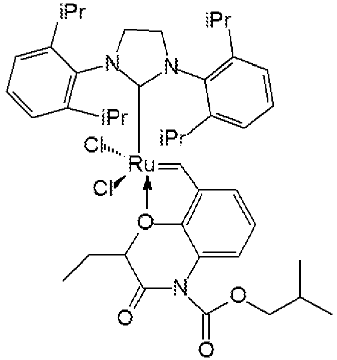
(14) Le catalyseur désigné par « M853-SIPr », de formule suivante : 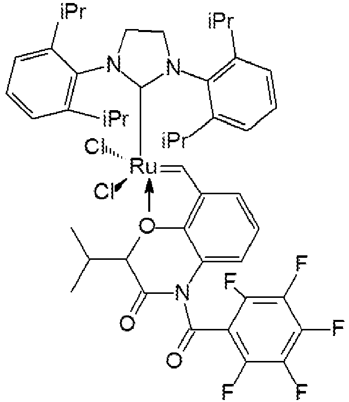
(15) Le catalyseur désigné par « M863-SIPr », de formule suivante : 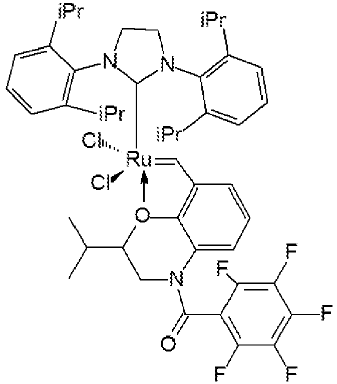
(16) Le catalyseur désigné par « Materia C711 », de formule suivante :

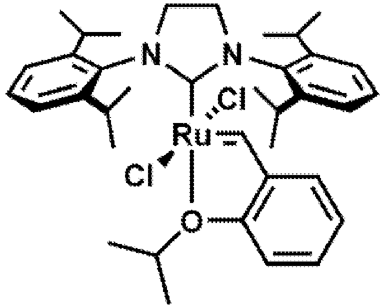

### EXEMPLES

Les exemples suivants illustrent l'invention sans la limiter.

Dans les exemples suivants, on utilise comme réactifs le 10-undécénoate de méthyle purifié et une oléfine qui est l'acrylonitrile et comme catalyseur le M71SiPr (commercialisé par Umicore) : 10,2mg sont dissous dans 21,6 g de toluène. 5 ml de cette solution sont ajoutés soit 2,02 mg de catalyseur.

Dans le procédé de l'exemple 1 selon l'invention, on utilise un module PF13F de DIC. Il s'agit d'une membrane dense en PTFE.

Dans le procédé des exemples comparatifs 2 et 3, on opère sans membrane.

On opère comme suit, et on obtient les résultats suivants.

### Description du montage

Le montage est composé d'un ballon de 600mL muni d'une arrivée d'azote, d'une sonde de température, d'un réfrigérant permettant la condensation et le recyclage au réacteur du solvant et de l'acrylonitrile notamment (surmonté d'un robinet et sortant sur un bulleur), d'un bain d'huile et d'une entrée pour réactifs et le catalyseur ajouté en continu.

Le milieu réactionnel est soutiré en continu par une pompe, placée en aval d'un échangeur de chaleur permettant de ramener la température du milieu réactionnel à 40 °C, et qui pousse le liquide au travers du module membranaire. Le liquide sortant du module passe aussi au travers d'un échangeur qui ramène le liquide à la température de la réaction, et le liquide est re-circulé dans le réacteur. Le module est maintenu sous un vide partiel de 60 kPa absolus. Le débit de circulation du liquide est de 200 ml/minute.

### Mode opératoire suivi :

- Purger le montage à l'azote. Puis couper l'azote lors du chargement de l'acrylonitrile
- Charger dans le réacteur 150 g de toluène
- Charger dans le réacteur :
   15 g de 10-undécénoate de méthyle purifié (par adsorption sur alumine)
   2,0 g d'acrylonitrile
- Chauffer à 110°C en laissant ouvert le robinet du réfrigérant, contrôler la température du milieu, (bain d'huile à 145°C)
- Peser 10,2 mg de catalyseur (M71SiPr, d'Umicore) dans un sabot en verre.

Le diluer dans 21,6 g de toluène dans un tube de Schlenk sous azote. 5 ml de la solution, soit 2,02 mg de catalyseur sont introduits dans la seringue qui est positionnée sur le pousse-seringue ; le catalyseur est préparé avant le chargement de l'acrylonitrile.
. Laisser le flux d'azote lors de la préparation du catalyseur et le couper lors du chargement. - Remplir d'environ 2,4 g une seringue avec l'acrylonitrile et la positionner sur le 2ème pousse seringue.

Prélever T=0
- Déclencher le début de la réaction T=0 avec l'ajout de catalyseur et d'acrylonitrile en continu pendant 2 heures.
- Faire un prélèvement à T=10 min, 20 min, 30min,.... et 120 min et faire une analyse GC de l'échantillon.
- Couper la chauffe, le bain du réfrigérant et laisser refroidir.

### Exemple 1 :

Au bout de 2 heures le rendement obtenu en nitrile ester est de 75 % en mole.

### Exemple comparatif 2 :

La même réaction est conduite en l'absence de module membranaire, toutes autres conditions égales par ailleurs.

Au terme des deux heures de réaction, le rendement est de 68 % en mole.

### Exemple comparatif 3 :

L'essai de l'exemple 2 est répété en utilisant une quantité double de catalyseur (M71SiPr).

Au terme des deux heures de réaction, le rendement est de 74 % en mole.

## Revendications

1. Procédé de métathèse de deux composés α-oléfiniques, **caractérisé en ce qu'**il comprend l'extraction de l'éthylène formé du milieu réactionnel au moyen d'au moins une membrane perméable aux gaz et imperméable aux liquides.

2. Procédé selon la revendication 1, **caractérisé en ce qu'**il est conduit dans un dispositif réactionnel comportant deux zones séparées par ladite au moins une membrane :
- une première zone, alimentée en réactifs et catalyseur, dans laquelle la réaction de métathèse en phase liquide est initiée et le milieu réactionnel liquide est mis en circulation au contact de la paroi constituée par la membrane, et
- une seconde zone, alimentée en un flux gazeux inerte vis-à-vis de la membrane et des constituants du milieu réactionnel de la première zone, éventuellement sous une pression légèrement inférieure à celle régnant dans la première zone ou sous la forme d'un balayage par un courant gazeux à débit suffisant, de façon à entraîner la migration par diffusion de l'éthylène dissous dans le milieu réactionnel de la première zone à la seconde zone.

3. Procédé selon la revendication 1 ou 2, **caractérisé en ce que** ladite au moins une membrane est d'épaisseur comprise dans la gamme de 5 à 1000 µm, de préférence de 5 à 700 µm, de préférence de 5 à 300 µm, et de préférence de 10 à 70 µm.

4. Procédé selon l'une quelconque des revendications 1 à 3, **caractérisé en ce que** ladite au moins une membrane comprend au moins une couche comportant au moins un polymère dense, et de préférence ladite au moins une membrane est uni-couche en polymère dense.

5. Procédé selon l'une quelconque des revendications 1 à 4, **caractérisé en ce que** ladite au moins une membrane est asymétrique et comporte une couche poreuse et une couche polymère dense, dans laquelle l'épaisseur totale de ladite membrane asymétrique est comprise dans la gamme de 5 à 300 µm, et de préférence de 10 à 70 µm, l'épaisseur de la membrane dense étant comprise dans la gamme de 0,01 à 10 µm et de préférence de 0,1 à 1 µm.

6. Procédé selon l'une quelconque des revendications 1 à 5, **caractérisé en ce que** ladite au moins une membrane a la forme d'une fibre creuse.

7. Procédé selon la revendication 6, dans lequel la fibre creuse a un diamètre extérieur compris dans la gamme de 50 à 1000 µm, de préférence de 150 à 700 µm et de manière plus préférée de 250 à 300 µm et un diamètre intérieur de la fibre compris dans la gamme de 30 à 300 µm.

8. Procédé selon l'une des revendications 6 ou 7 **caractérisé en ce qu'**il est conduit en présence d'un faisceau de fibres creuses immergé dans le réacteur de métathèse et au moyen duquel l'éthylène est soutiré et extrait par balayage gazeux, de préférence par balayage gazeux à l'intérieur de la fibre, ledit balayage gazeux ayant de préférence une pression légèrement inférieure à celle du réacteur.

9. Procédé selon l'une des revendications 1 à 7, **caractérisé en ce que** la réaction de métathèse est conduite dans un réacteur connecté à un dispositif de séparation membranaire comportant au moins un module d'extraction de l'éthylène au moyen duquel au moins un flux liquide issu du milieu réactionnel est adressé au dispositif de séparation membranaire d'extraction de l'éthylène et au moins un flux liquide sortant dudit dispositif est retourné dans le réacteur pour la poursuite de la réaction.

10. Procédé selon la revendication 9, dans lequel on utilise en outre deux échangeurs thermiques, le premier en amont du dispositif de séparation membranaire pour abaisser la température du flux liquide au seuil de fonctionnement du séparateur membranaire et le second en aval du dispositif de séparation membranaire pour remonter la température du flux liquide sortant au niveau de celle du milieu réactionnel du réacteur.

11. Procédé selon l'une des revendications précédentes, **caractérisé en ce que** les polymères utilisables dans la composition de ladite au moins une membrane sont choisis parmi les polymères oléophobes tels que les polyoléfines telles que le polyéthylène, le polypropylène, le poly-3-méthylbutène-1 et le poly-4-méthylpentène-1 (PMP); les polymères vinyliques tels que le polystyrène, le PMMA (polyméthyl-méthacrylate) ; les polysulfones ; les polymères fluorés ou chlorés tels que le polyvinylidène fluoré (PVDF), le polytétrafluoroéthylène (PTFE), les copolymères fluorovinyléthylène / tétrafluoroéthylène, le polychloroprène (PCP) ; les polyamides tels que le nylon 6, le nylon 66 et le nylon 12 ; les polyesters tels que le polyéthylènetéréphtalate, le polybutènetéréphtalate et le polyéthylène-2,6-naphtalate ; les polycarbonates tels que le poly-4,4'-dihydroxydiphénil-2,2-propane carbonate ; les polyéthers tels que le poly-oxyméthylène et le polyméthylène sulfure ; les poly-phénylènes chalcogénures tels que les polythioéther, polyphénylène oxyde et polyphénylène sulfure ; les polyéther éther cétones (PEEK), les polyéther cétone cétone (PEKK), les silicones tels que le polyvinyltriméthylsiloxane (PVTMS), le polydiméthylsiloxane (PDMS), le perfluoroalkoxy (PFA).

12. Procédé selon la revendication 11, **caractérisé en ce que** la membrane dense comprend au moins un polymère choisi parmi le polytétrafluoroéthylène (PTFE), le perfluoroalkoxy (PFA), et leurs mélanges.

13. Procédé selon l'une des revendications précédentes, **caractérisé en ce que** la réaction de métathèse est conduite en milieu liquide en présence d'un catalyseur à une température comprise dans la gamme de 20 à 160 °C et de préférence de 20 à 120 °C et sous une pression comprise dans la gamme de 1 à 30 bars et de préférence dans la gamme de 1 à 10 bars, et de façon plus préférée à la pression atmosphérique lorsque la température d'ébullition des réactifs mis en jeu le permet.

14. Procédé selon l'une des revendications précédentes, **caractérisé en ce que** la réaction est conduite en présence d'au moins un solvant choisi parmi le toluène, les xylènes ou le dichlorométhane.

15. Procédé selon l'une des revendications précédentes, **caractérisé en ce que** la réaction est conduite en présence d'un catalyseur de métathèse de type ruthénium-carbène, ledit catalyseur ruthénium-carbène étant choisi de préférence parmi les catalyseurs chargés ou non-chargés de formule générale :
(X₁)ₐ(X₂)_{b}Ru(carbène C)(L₁)_{c}(L₂)_{d}(L₃)ₑ
dans laquelle :
- a, b, c, d et e sont des nombres entiers, identiques ou différents, avec a et b égaux à 0, 1 ou 2 ; c, d et e égaux à 0, 1, 2, 3 ou 4;
- X₁ et X₂, identiques ou différents, représentent chacun un ligand mono- ou multi-chélatant, chargé ou non ; à titre d'exemples, on pourra citer les
- phénolates, les amidures, le tosylate, l'hexafluorophosphate, le tétrafluoroborate, le bis-triflylamidure, un alkyle, le tétraphénylborate et dérivés ; X₁ ou X₂ peuvent être liés à L₁ ou L₂ ou au carbène C de façon à former un ligand bidenté ou chélate sur le ruthénium ; et
- L₁, L₂ et L₃ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique ; L₁, L₂ ou L₃ peuvent être liés au carbène C de façon à former un ligand bidenté ou chélate, ou tridenté.
Le carbène C est représenté par la formule générale : CR₁R₂ pour laquelle R₁ et R₂ sont des groupes identiques ou différents tels que l'hydrogène ou tout autre groupe hydrocarboné, fonctionnalisé ou non, de type saturé, insaturé, cyclique, aromatique, branché et/ou linéaire.

16. Procédé selon la revendication 15, **caractérisé en ce que** le catalyseur est choisi parmi les complexes du ruthénium alkylidènes, benzylidène, benzylidène éther ou cumylènes tels que les vinylidènes Ru=C=CHR ou allénylidènes Ru=C=C=CR₁R₂ ou indénylidènes, un groupe fonctionnel (permettant d'améliorer la rétention du complexe du ruthénium dans un liquide ionique) pouvant être greffé sur au moins l'un des ligands X₁, X₂, L₁, L₂, ou sur le carbène C, ce groupe fonctionnel pouvant être chargé ou non chargé tel qu'un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

## Patentansprüche

1. Verfahren zur Metathese von zwei α-Olefinverbindungen, **dadurch gekennzeichnet, dass** es die Extraktion des gebildeten Ethylens aus dem Reaktionsmedium mit Hilfe mindestens einer gasdurchlässigen und flüssigkeitsundurchlässigen Membran umfasst.

2. Verfahren nach Anspruch 1, **dadurch gekennzeichnet, dass** es in einer Reaktionsvorrichtung mit zwei durch die mindestens eine Membran getrennten Zonen durchgeführt wird:
- einer mit Reagenzien und Katalysator gespeisten ersten Zone, in der die Flüssigphasen-Metathesereaktion initiiert wird und das flüssige Reaktionsmedium in Kontakt mit der aus der Membran bestehenden Wand in Umlauf gebracht wird, und
- einer zweiten Zone, die mit einem Strom von gegenüber der Membran und den Bestandteilen des Reaktionsmediums der ersten Zone inerten Gas gespeist wird, gegebenenfalls unter einem etwas unterhalb des in der ersten Zone herrschenden Drucks liegenden Druck oder in Form einer Spülung mit einem Gasstrom mit ausreichender Durchflussrate, um die Migration des in dem Reaktionsmedium der ersten Zone gelösten Ethylens in die zweite Zone durch Diffusion herbeizuführen.

3. Verfahren nach Anspruch 1 oder 2, **dadurch gekennzeichnet**, das die mindestens eine Membran eine Dicke im Bereich von 5 bis 1000 µm, vorzugsweise von 5 bis 700 µm, vorzugsweise von 5 bis 300 µm und vorzugsweise von 10 bis 70 µm aufweist.

4. Verfahren nach einem der Ansprüche 1 bis 3, **dadurch gekennzeichnet, dass** die mindestens eine Membran mindestens eine Schicht umfasst, die mindestens ein dichtes Polymer umfasst, und vorzugsweise die mindestens eine Membran eine einzige Schicht aus dichtem Polymer ist.

5. Verfahren nach einem der Ansprüche 1 bis 4, **dadurch gekennzeichnet, dass** die mindestens eine Membran asymmetrisch ist und eine poröse Schicht und eine dichte Polymerschicht umfasst, wobei die Gesamtdicke der asymmetrischen Membran im Bereich von 5 bis 300 µm und vorzugsweise von 10 bis 70 µm liegt, wobei die Dicke der dichten Membran im Bereich von 0,01 bis 10 µm und vorzugsweise von 0,1 bis 1 µm liegt.

6. Verfahren nach einem der Ansprüche 1 bis 5, **dadurch gekennzeichnet, dass** die mindestens eine Membran in Form einer Hohlfaser vorliegt.

7. Verfahren nach Anspruch 6, wobei die Hohlfaser einen Außendurchmesser im Bereich von 50 bis 1000 µm, vorzugsweise von 150 bis 700 µm und weiter bevorzugt von 250 bis 300 µm und einen Innendurchmesser der Faser im Bereich von 30 bis 300 µm aufweist.

8. Verfahren nach Anspruch 6 oder 7, **dadurch gekennzeichnet, dass** es in Gegenwart eines Hohlfaserbündels durchgeführt wird, das in den Metathesereaktor eingetaucht ist und wodurch das Ethylen abgezogen und extrahiert wird, und zwar durch Gasspülung, vorzugsweise durch Gasspülung im Inneren der Faser, wobei die Gasspülung vorzugsweise einen etwas unter dem Reaktordruck liegenden Druck aufweist.

9. Verfahren nach einem der Ansprüche 1 bis 7, **dadurch gekennzeichnet, dass** die Metathesereaktion in einem Reaktor durchgeführt wird, der mit einer Membrantrennvorrichtung verbunden ist, die mindestens ein Ethylenextraktionsmodul umfasst, wodurch mindestens ein flüssiger Strom aus dem Reaktionsmedium der Membrantrennvorrichtung zur Extraktion von Ethylen zugeführt wird und mindestens ein flüssiger Strom aus der Vorrichtung zur Weiterführung der Reaktion wieder in den Reaktor zurückgeführt wird.

10. Verfahren nach Anspruch 9, bei dem man außerdem zwei Wärmetauscher verwendet, den ersten stromaufwärts der Membrantrennvorrichtung zur Verringerung der Temperatur des flüssigen Stroms auf den Betriebsschwellenwert des Membranseparators und den zweiten stromabwärts der Membrantrennvorrichtung zum Wiedererhöhen der Temperatur des austretenden flüssigen Stroms auf das Temperaturniveau des Reaktionsmediums des Reaktors.

11. Verfahren nach einem der vorhergehenden Ansprüche, **dadurch gekennzeichnet, dass** die in der Zusammensetzung der mindestens einen Membran verwendbaren Polymere aus oleophoben Polymeren wie Polyolefinen wie Polyethylen, Polypropylen, Poly-3-methyl-1-buten und Poly-4-methyl-1-penten (PMP); Vinylpolymeren wie Polystyrol, PMMA (Polymethylmethacrylat); Polysulfonen; fluorierten oder chlorierten Polymeren wie Polyvinylidenfluorid (PVDF), Polytetrafluorethylen (PTFE), Fluorvinylethylen/Tetrafluorethylen-Copolymeren, Polychloropren (PCP); Polyamiden wie Polyamid 6, Polyamid 66 und Polyamid 12; Polyestern wie Polyethylenterephthalat, Polybutenterephthalat und Polyethylen-2,6-naphthalat; Polycarbonaten wie Poly-4,4'-dihydroxydiphenyl-2,2-propancarbonat; Polyethern wie Polyoxymethylen und Polymethylensulfid; Polyphenylenchalcogeniden wie Polythioether, Polyphenylenoxid und Polyphenylensulfid; Polyetheretherketonen (PEEK), Polyetherketonketon (PEKK), Silikonen wie Polyvinyltrimethylsiloxan (PVTMS), Polydimethylsiloxan (PDMS), Perfluoralkoxy (PFA) ausgewählt sind.

12. Verfahren nach Anspruch 11, **dadurch gekennzeichnet, dass** die dichte Membran mindestens ein Polymer umfasst, das aus Polytetrafluorethylen (PTFE), Perfluoralkoxy (PFA) und Mischungen davon ausgewählt ist.

13. Verfahren nach einem der vorhergehenden Ansprüche, **dadurch gekennzeichnet, dass** die Metathesereaktion in flüssigem Medium in Gegenwart eines Katalysators bei einer Temperatur im Bereich von 20 bis 160 °C und vorzugsweise von 20 bis 120 °C und unter einem Druck im Bereich von 1 bis 30 bar und vorzugsweise im Bereich von 1 bis 10 bar und weiter bevorzugt bei Normaldruck, wenn der Siedepunkt der beteiligten Reagenzien dies zulässt, durchgeführt wird.

14. Verfahren nach einem der vorhergehenden Ansprüche, **dadurch gekennzeichnet, dass** die Reaktion in Gegenwart mindestens eines aus Toluol, Xylolen oder Dichlormethan ausgewählten Lösungsmittels durchgeführt wird.

15. Verfahren nach einem der vorhergehenden Ansprüche, **dadurch gekennzeichnet, dass** die Reaktion in Gegenwart eines Metathesekatalysators vom Ruthenium-Carben-Typ durchgeführt wird, wobei der Ruthenium-Carben-Katalysator vorzugsweise aus den geladenen oder ungeladenen Katalysatoren der folgenden allgemeinen Formel ausgewählt wird:
(X₁)ₐ(X₂)_{b}Ru(Carben C)(L₁)_{c}(L₂)_{d}(L₃)ₑ,
wobei
- a, b, c, d und e gleiche oder verschiedene ganze Zahlen sind, wobei a und b gleich 0, 1 oder 2 sind; c, d und e gleich 0, 1, 2, 3 oder 4 sind;
- X₁ und X₂ gleich oder verschieden sind und jeweils für einen geladenen oder ungeladenen Mono- oder Multichelatliganden stehen; als Beispiele seien Phenolate, Amide, Tosylat, Hexafluorophosphat, Tetrafluoroborat, Bis(triflyl)amid, ein Alkyl, Tetraphenylborat und Derivate genannt; X₁ oder X₂ an L₁ oder L₂ oder an das Carben C gebunden sein können, so dass sich ein zweizähniger Ligand oder Chelatligand am Ruthenium ergibt; und
- L₁, L₂ und L₃ gleich oder verschieden sind und für elektronenliefernde Liganden wie Phosphin, Phosphit, Phosphonit, Phosphinit, Arsin, Stilbin, ein Olefin oder einen Aromaten, eine Carbonylverbindung, einen Ether, einen Alkohol, ein Amin, ein Pyridin oder Derivat, ein Imin, einen Thioether oder ein heterocyclisches Carben stehen; L₁, L₂ und L₃ an das Carben C gebunden sein können, so dass sich ein zweizähniger Ligand oder Chelatligand oder ein dreizähniger Ligand ergibt.
Das Carben C wird durch die allgemeine Formel CR₁R₂ wiedergegeben, in der R₁ und R₂ gleiche oder verschiedene Gruppen sind wie Wasserstoff oder eine beliebige andere funktionalisierte oder nicht funktionalisierte Kohlenwasserstoffgruppe vom gesättigten, ungesättigten, cyclischen, aromatischen, verzweigten und/oder linearen Typ.

16. Verfahren nach Anspruch 15, **dadurch gekennzeichnet, dass** der Katalysator aus Alkyliden-, Benzyliden-, Benzylyidenether- oder Cumylen-Ruthenium-Komplexen wie den Vinylidenen Ru=C=CHR oder Allenylidenen Ru=C=C=CR₁R₂ oder Indenylidenen ausgewählt wird, wobei eine funktionelle Gruppe (zur Verbesserung der Retention des Rutheniumkomplexes in einer ionischen Flüssigkeit) auf mindestens einen der Liganden X₁, X₂, L₁, L₂ oder auf das Carben C aufgepfropft sein kann, wobei diese funktionelle Gruppe geladen oder ungeladen sein kann, wie ein Ester, ein Ether, ein Thiol, eine Säure, ein Alkohol, ein Amin, ein Stickstoff-Heterocyclus, ein Sulfonat, ein Carboxylat, ein quartäres Ammonium, ein Guanidinium, ein quartäres Phosphonium, ein Pyridinium, ein Imidazolium, ein Morpholinium oder ein Sulfonium.

## Claims

1. Process for the metathesis of two α-olefinic compounds, **characterized in that** it comprises the extraction of the formed ethylene from the reaction medium by means of at least one membrane that is permeable to gases and impermeable to liquids.

2. Process according to Claim 1, **characterized in that** it is performed in a reaction device comprising two zones separated by said at least one membrane:
- a first zone, fed with reagents and catalyst, in which the liquid-phase metathesis reaction is initiated and the liquid reaction medium is placed in circulation in contact with the wall constituted by the membrane, and
- a second zone, fed with a stream of gas that is inert toward the membrane and the constituents of the reaction medium of the first zone, optionally under a pressure slightly lower than that prevailing in the first zone or in the form of a flush with a gas stream at a sufficient flow rate, so as to bring about the migration by diffusion of the ethylene dissolved in the reaction medium from the first zone to the second zone.

3. Process according to Claim 1 or 2, **characterized in that** said at least one membrane has a thickness in the range from 5 to 1000 µm, preferably from 5 to 700 µm, preferably from 5 to 300 µm and preferably from 10 to 70 µm.

4. Process according to any one of Claims 1 to 3, **characterized in that** said at least one membrane comprises at least one layer comprising at least one dense polymer, and preferably said at least one membrane is a single layer made of dense polymer.

5. Process according to any one of Claims 1 to 4, **characterized in that** said at least one membrane is asymmetrical and comprises a porous layer and a dense polymer layer, in which the total thickness of said asymmetrical membrane is in the range from 5 to 300 µm and preferably from 10 to 70 µm, the thickness of the dense membrane being in the range from 0.01 to 10 µm and preferably from 0.1 to 1 µm.

6. Process according to any one of Claims 1 to 5, **characterized in that** said at least one membrane is in the form of a hollow fibre.

7. Process according to Claim 6, in which the hollow fibre has an outside diameter in the range from 50 to 1000 µm, preferably from 150 to 700 µm and more preferably from 250 to 300 µm and an inside diameter of the fibre in the range from 30 to 300 µm.

8. Process according to either of Claims 6 and 7, **characterized in that** it is performed in the presence of a bundle of hollow fibres immersed in the metathesis reactor and by means of which ethylene is withdrawn and extracted by flushing with gas, preferably by flushing with gas inside the fibre, said flushing with gas preferably having a pressure slightly below that of the reactor.

9. Process according to one of Claims 1 to 7, **characterized in that** the metathesis reaction is performed in a reactor connected to a membrane separation device comprising at least one ethylene extraction module by means of which at least one liquid flow derived from the reaction medium is sent to the membrane separation device for extracting ethylene and at least one liquid flow exiting said device is returned into the reactor for the continuation of the reaction.

10. Process according to Claim 9, in which two heat exchangers are also used, the first upstream of the membrane separation device for lowering the temperature of the liquid flow to the operating threshold of the membrane separator, and the second downstream of the membrane separation device for raising the temperature of the exiting liquid flow to the temperature level of the reaction medium of the reactor.

11. Process according to one of the preceding claims, **characterized in that** the polymers that may be used in the composition of said at least one membrane are chosen from oleophobic polymers such as polyolefins such as polyethylene, polypropylene, poly-3-methylbutene-1 and poly(4-methyl-1-pentene) (PMP); vinyl polymers such as polystyrene, PMMA (polymethyl methacrylate); polysulfones; fluorinated or chlorinated polymers such as polyvinylidene fluoride (PVDF), polytetrafluoroethylene (PTFE), fluorovinylethylene/tetrafluoroethylene copolymers, polychloroprene (PCP); polyamides such as nylon 6, nylon 66 and nylon 12; polyesters such as polyethylene terephthalate, polybutene terephthalate and polyethylene-2,6-naphthalate; polycarbonates such as poly-4,4'-dihydroxydiphenyl-2,2-propane carbonate; polyethers such as polyoxymethylene and polymethylene sulfide; polyphenylene chalcogenides such as polythioether, polyphenylene oxide and polyphenylene sulfide; polyether ether ketones (PEEK), polyether ketone ketone (PEKK), silicones such as polyvinyltrimethylsiloxane (PVTMS), polydimethylsiloxane (PDMS), perfluoroalkoxy (PFA).

12. Process according to Claim 11, **characterized in that** the dense membrane comprises at least one polymer chosen from polytetrafluoroethylene (PTFE), perfluoroalkoxy (PFA), and mixtures thereof.

13. Process according to one of the preceding claims, **characterized in that** the metathesis reaction is performed in liquid medium in the presence of a catalyst at a temperature in the range from 20 to 160°C and preferably from 20 to 120°C and at a pressure in the range from 1 to 30 bar and preferably in the range from 1 to 10 bar, and more preferably at atmospheric pressure when the boiling point of the reagents involved permits it.

14. Process according to one of the preceding claims, **characterized in that** the reaction is performed in the presence of at least one solvent chosen from toluene, xylenes and dichloromethane.

15. Process according to one of the preceding claims, **characterized in that** the reaction is performed in the presence of a metathesis catalyst of ruthenium-carbene type, said ruthenium-carbene catalyst preferably being chosen from the charged or uncharged catalysts of general formula:
(X₁)ₐ(X₂)_{b}Ru(carbene C)(L₁)_{c}(L₂)_{d}(L₃)ₑ
in which:
- a, b, c, d and e are integers, which may be identical or different, with a and b equal to 0, 1 or 2; c, d and e equal to 0, 1, 2, 3 or 4;
- X₁ and X₂, which may be identical or different, each represent a charged or uncharged and mono-chelating or polychelating ligand; examples that may be mentioned include
- phenoxides, amides, tosylate, hexafluorophosphate, tetrafluoroborate, bis(triflyl)amide, an alkyl, tetraphenylborate and derivatives; X₁ or X₂ may be linked to L₁ or L₂ or to the carbene C so as to form a bidentate ligand or chelate on the ruthenium; and
- L₁, L₂ and L₃, which may be identical or different, are electron-donating ligands, such as phosphine, phosphite, phosphonite, phosphinite, arsine, stilbene, an olefin or an aromatic compound, a carbonyl compound, an ether, an alcohol, an amine, a pyridine or derivative, an imine, a thioether, or a heterocyclic carbene; L₁, L₂ or L₃ can be bonded to the carbene C so as to form a bidentate or chelate ligand, or a tridentate ligand.
The carbene C is represented by the general formula: CR₁R₂ for which R₁ and R₂ are groups which may be identical or different, such as hydrogen or any other functionalized or non-functionalized hydrocarbon-based group of saturated, unsaturated, cyclic, aromatic, branched and/or linear type.

16. Process according to Claim 15, **characterized in that** the catalyst is chosen from alkylidene, benzylidene, benzylidene ether or cumylene ruthenium complexes such as the vinylidenes Ru=C=CHR or allenylidenes Ru=C=C=CR₁R₂ or indenylidenes, a functional group (for improving the retention of the ruthenium complex in an ionic liquid) possibly being grafted onto at least one of the ligands X₁, X₂, L₁, L₂, or onto the carbene C, this functional group possibly being charged or uncharged such as an ester, an ether, a thiol, an acid, an alcohol, an amine, a nitrogenous heterocycle, a sulfonate, a carboxylate, a quaternary ammonium, a guanidinium, a quaternary phosphonium, a pyridinium, an imidazolium, a morpholinium or a sulfonium.
